# EUROPEAN PATENT SPECIFICATION

(11) **EP 4 389 763 B1**
(45) Date of publication and mention of the grant of the patent: **28.01.2026**
(21) Application number: 22866291.2
(22) Date of filing: 03.08.2022
(51) Int. Cl.: C07K 14/50, A61K 38/18, C12N 15/63, C12N 5/10, A61P 3/10, A61P 3/04, A61P 3/06

(54) **FGF21 MUTANT PROTEIN AND USE THEREOF**
FGF21-MUTANTES PROTEIN UND VERWENDUNG DAVON
PROTÉINE MUTANTE FGF21 ET SON UTILISATION

(30) Priority: 08.09.2021 CN 202111048898
(43) Date of publication of application: 26.06.2024
(73) Proprietor: Leto Laboratories Co., Ltd, Beijing 100094 (CN)
(72) Inventor: GAO, Xiang, Beijing 100094 (CN); ZHAO, Yao, Beijing 100094 (CN); ZHANG, Jianjun, Beijing 100094 (CN); GAO, Zhan, Beijing 100094 (CN); ZHANG, Yu, Beijing 100094 (CN)
(74) Representative: Bayramoglu et al.
(86) International application number: PCT/CN2022/110072
(87) International publication number: WO 2023/035817

(56) References cited:
- WO-A1-2016/102562
- WO-A1-2017/059371
- WO-A2-2010/042747
- WO-A2-2010/129600
- WO-A2-2016/065326
- CN-A- 107 056 925
- CN-A- 107 188 950
- CN-A- 108 602 869
- CN-A- 110 128 525
- CN-A- 113 366 024
- SHANAKA STANISLAUS ET AL: "A Novel Fc-FGF21 With Improved Resistance to Proteolysis, Increased Affinity Toward [beta]-Klotho, and Enhanced Efficacy in Mice and Cynomolgus Monkeys", ENDOCRINOLOGY, vol. 158, no. 5, 26 January 2017 (2017-01-26), US, pages 1314 - 1327, XP055682577, ISSN: 0013-7227, DOI: 10.1210/en.2016-1917

## Description

### TECHNICAL FIELD

The present disclosure relates to a fibroblast growth factor 21 (FGF21) mutant protein and use thereof, and belongs to the field of biotechnologies.

### BACKGROUND

Human FGF21 is a polypeptide composed of 209 amino acids. The N-terminus of the FGF21 protein includes a signal peptide composed of 28 amino acids and the mature FGF21 produced after cleavage of the signal peptide is composed of 181 amino acids (SEQ ID NO: 1).

FGF21 is expressed in a variety of human organs and tissues, such as the liver, pancreas, and adipose tissue. In the human body, FGF21 is secreted into the blood circulation and can induce a variety of signaling pathways and functional activities in the liver, pancreas, and adipose tissue, allowing the physiological functions of regulating glycolipid metabolism and protecting pancreatic β cells. Studies have shown that injection of the FGF21 protein into obese mice produced by diet induction or genetic manipulation can significantly reduce the body weight and blood glucose level and can also significantly reduce the serum triglyceride content of the obese mice. Further studies have shown that FGF21 can improve insulin sensitivity of the liver to alleviate glucose intolerance. Studies in mammals close to humans have shown that FGF21 can reduce the body weight and body fat content of the experimental animals in a dose-dependent manner. For example, it has been found that the administration of FGF21 to diabetic rhesus monkeys can significantly reduce fasting plasma glucose and triglyceride levels in the diabetic rhesus monkeys. In addition, FGF21 can also activate signaling pathways for external secretion of pancreatic cells and hepatic cells to inhibit the output of hepatic glycogens.

FGF21 is a member of the fibroblast growth factor (FGF) family. However, unlike most FGFs, which have a broad-spectrum ability to promote mitosis, FGF21 does not have a significant ability to promote cell proliferation. Studies have shown that FGF21 transgenic mice undergo no abnormal conditions such as tumors and tissue hyperplasia throughout their life cycles. In addition, pharmacokinetic and drug safety experiments have shown that FGF21 does not cause hypoglycemia when applied at a dose higher than the pharmacological dose, indicating that FGF21 is a potential ideal drug for treating diseases such as diabetes and obesity.

Unlike most FGF family members, FGF21 activates a downstream signaling pathway by both fibroblast growth factor receptors (FGFRs) and coreceptor β-klotho. Neither β-klotho nor FGFR alone can activate the FGF21 signaling. Structural biology studies have shown that the structure of FGF21 can be divided into two parts: the N-terminal domain is mainly related to FGFR binding, while the C-terminal domain is a relatively flexible sequence and is related to β-klotho binding. Only after FGF21 forms a ternary complex with β-klotho and FGFR, can it activate a downstream related signaling pathway to exert its biological effect.

The physiological functions of FGF21 in controlling blood glucose and reducing body weight have brought hope for the treatment of related diseases. However, wild-type FGF21 shows disadvantages such as being easily hydrolyzed by proteases, having a too small molecular weight, and having a half-life of merely 0.5 h to 2 h, thus making wild-type FGF21 unsuitable for direct use as a drug. At the same time, the flexible region at the C-terminus of the FGF21 protein that is responsible for binding to the β-klotho receptor is easily degraded by fibroblast activation protein (FAP), with its major degradation site around Pro171. When P171 is cleaved by FAP, the FGF21 molecule can no longer bind to the coreceptor β-klotho, resulting in inactivation of the FGF21 molecule. Faced with this difficulty, the medical and pharmaceutical industry has improved the half-life of FGF21 by preparing a long-acting fusion protein through site-directed mutagenesis of an amino acid at the enzyme cleavage site, or by linking polyethylene glycol (PEG), fatty acid chain, etc. to the polypeptide skeleton.

PCT publications WO 2016/065326 A2, WO 2010/042747 A2, WO 2010/129600 A2, WO 2016/102562 A1, WO 2017/059371 A1 and Shanka Stanislaus et al: "A Novel Fc-FGF21 With Improved Resistance to Proteolysis, Increased Affinity Toward [beta]-Klotho, and Enhanced Efficacy in Mice and Cynomolgus Monkeys", Endocrinology, vol. 158, no. 5, 26 January 2017 (2017-01-26), pages 1314-1327, disclose different FGF-21 variants with improved stability or increased half-life. US8,034,770B2 discloses some mutants based on the polypeptide sequence of human wild-type FGF21 in which P171G or P171A can effectively slow down the hydrolysis of the C-terminus of the FGF21 protein by FAP. However, according to either the report of this patent or the *in vitro* experiments of the present disclosure, P171G still may be inactivated by FAP hydrolysis to a certain extent. Therefore, the discovery of a novel, more effective mutant to attenuate the hydrolysis by FAP is of great significance for further improving the druggability of the FGF21 protein.

### SUMMARY

A native wild-type human FGF21 protein has an amino acid sequence shown in SEQ ID NO: 1, and a proline residue (Pro) at position 171 of the wild-type human FGF21 protein is an enzyme cleavage site of FAP. An objective of the present disclosure is to reduce a hydrolysis rate of FGF21 by changing an enzyme cleavage site of wild-type FGF21 and/or a structure nearby to prolong an *in vivo* half-life and efficacy of FGF21.

To allow the above objective the present disclosure provides the solutions presented in the appended claims.

A C-terminus of the FGF21 mutant protein provided by the present disclosure can resist the enzymatic degradation of FAP and maintain an activity of binding to a receptor β-klotho (KLB), which can prolong an *in vivo* half-life and efficacy of the FGF21 mutant protein. The FGF21 mutant in the specific embodiments of the present disclosure has a stronger ability to slow down the hydrolysis of FAP than the mutant P171G.

All amino acid sequence numbers involved in the present disclosure are based on wild-type FGF21 (SEQ ID NO: 1). In SEQ ID NO: 1, a first amino acid position is numbered 1, a second amino acid position is numbered 2, and so on.

In order to make the objectives, features, and effects of the present disclosure fully understood, the concepts, specific structures, and technical effects of the present disclosure are further described below in conjunction with accompanying drawings.

### BRIEF DESCRIPTION OF THE DRAWINGS

FIG. 1 is a schematic structural diagram of molecular simulation of a disulfide bond formed at two sides of a proline residue at position 171 (with 170C/173C as an example, SEQ ID NO: 5); and
FIG. 2 is a sodium dodecyl-sulfate polyacrylamide gel electrophoresis (SDS-PAGE) pattern of the protein in Example 2 after expression and purification, where M is a marker.

### DETAILED DESCRIPTION OF THE EMBODIMENTS

### Example 1 Construction and expression of a recombinant expression vector

### 1.1 Construction of an expression plasmid

GENEWIZ was entrusted to synthesize genes that could express proteins numbered 1414, 1407, 1408, 1409, 1410, 1411, 1412, and 1413, respectively, and the genes each were cloned into a pET21b vector and then transformed into a BL21 (DE3) strain.

An FGF21 (121Q, 168L) mutant 1414 has an amino acid sequence shown in SEQ ID NO: 2, and mutation sites 121Q and 168L are intended to weaken a deamination reaction at position 121 and an oxidation reaction at position 168 and do not affect an activity or other functions of the protein (as disclosed in WO2011154349A2 and CN113728013A). Therefore, in the example, the mutant 1414 is adopted as a control, and experimental results of the mutant 1414 represent experimental results of wild-type FGF21. In addition, an alanine residue is added before a histidine residue at position 1 of wild-type FGF21 to remove a start codon of methionine for prokaryotic expression. An FGF21 (P171G) mutant 1407 has an amino acid sequence shown in SEQ ID NO: 3; an FGF21 (Del-P171) mutant 1408 has an amino acid sequence shown in SEQ ID NO: 4; an FGF21 (170C/Ins-172G/173C) mutant 1409 has an amino acid sequence shown in SEQ ID NO: 5; an FGF21 (170C/172C) mutant 1410 has an amino acid sequence shown in SEQ ID NO: 6; an FGF21 (170C/173C) mutant 1411 has an amino acid sequence shown in SEQ ID NO: 7; an FGF21 (170C/Ins-172A/173C) mutant 1412 has an amino acid sequence shown in SEQ ID NO: 8; and an FGF21 (170C/Ins-172S/173C) mutant 1413 has an amino acid sequence shown in SEQ ID NO: 9.

### 1.2 Expression

A seed culture cultivated overnight was transferred into an Erlenmeyer flask including 500 mL a Kana-resistant TB medium in a volume ratio of 1:50 with an initial OD₆₀₀ of about 0.1, and cultivated at 37°C and 220 rpm until an OD₆₀₀ was 2.0; and isopropyl β-D-thiogalactoside (IPTG) was added (final concentration: 0.5 mmol/L), the bacterial solution was further cultivated overnight at 37°C and 220 rpm, and resulting bacteria were collected. The protein was expressed in a form of an inclusion body (IB).

### Example 2 Renaturation and purification of FGF21 and mutant proteins thereof

### 2.1 Denaturation and dissolution of IBs

IBs obtained through cell disruption and washing in Example 1 were dissolved with 8 M urea and 10 mM DTT. The dissolution of IBs was conducted at room temperature for 4 h.

### 2.2 Renaturation

An IB solution obtained after the dissolution above was added to a renaturation solution including 2 M urea, 10 mM cysteine, and a 20 mM Tris-Cl buffer at a pH of 8.0, and incubated overnight at room temperature. During the renaturation, continuous stirring was required at a rotational speed of 200 rpm.

### 2.3 Purification of FGF21 and mutant proteins thereof by hydrophobic interaction chromatography Phenyl

NaCl at a final concentration of 2 M and a Tris-Cl buffer at a final concentration of 20 mM were added, and then flowed through a hydrophobic chromatography phenyl column (purchased from GE), and then gradient elution was conducted with NaCl solutions from 2 M to 0 M. According to SDS-PAGE, collection tubes with a target protein were selected, and target protein products in these collection tubes were pooled and stored. This step could capture a target protein and remove some impurity proteins.

### 2.4 Purification of FGF21 and mutant proteins thereof by ion-exchange chromatography Source 30Q

A pooled protein product obtained after the hydrophobic interaction chromatography was diluted 10-fold and then subjected to ion-exchange chromatography Source 30Q, where gradient elution was conducted with NaCl solutions from 0 M to 1.0 M. This step could further remove nucleic acids, some protein polymers, and impurity proteins.

### 2.5 Fine purification of FGF21 and mutant proteins thereof by a molecular sieve

A pooled protein product obtained after the ion-exchange chromatography Source 30Q was concentrated and then finely purified with a molecular sieve Superdex 200 pg with phosphate buffered saline (PBS) as a buffer. A purified protein obtained in this step was determined as a final sample.

Purification information is shown in Table 1, and it can be seen that all proteins have an electrophoresis-pure purity.

**Table 1 Concentrations of purified FGF21 and mutants**

| Protein No. | Concentration (mg/mL) |
|---|---|
| 1414 | 3.2 |
| 1407 | 2.9 |
| 1408 | 3.3 |
| 1409 | 1.6 |
| 1410 | 1.78 |
| 1411 | 1.25 |
| 1412 | 2.2 |
| 1413 | 2.5 |

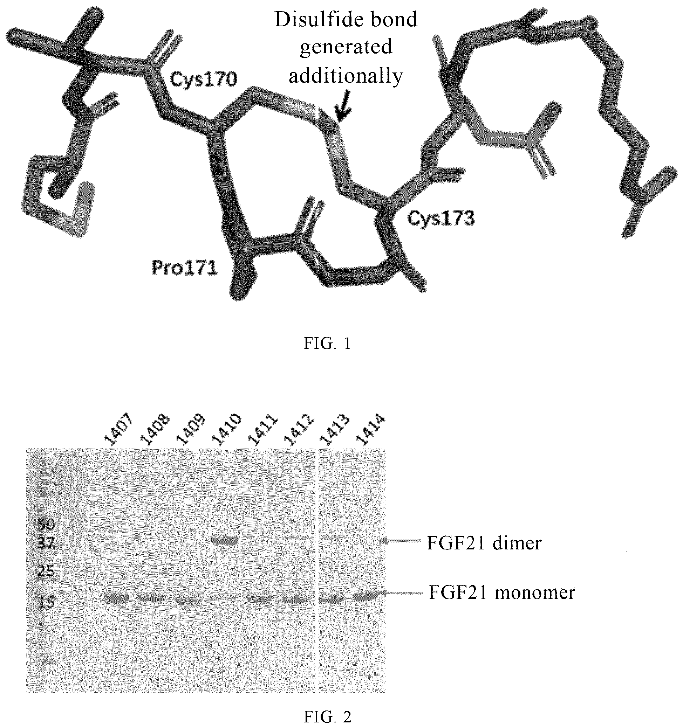

It can be seen from the SDS-PAGE analysis results of the final FGF21 mutant 1414 and proteins obtained from purification of the mutants (FIG. 2) that the FGF21 mutant 1414 and the mutants 1407, 1408, and 1409 all exist in a monomer form, where a disulfide bond additionally introduced in the FGF21 mutant 1409 has completely become an intramolecular disulfide bond, and does not improperly become an intermolecular disulfide bond to cause the generation of an FGF21 dimer. A large number of dimers are produced in the FGF21 mutant 1410, indicating that disulfide bonds additionally introduced in this mutant cannot properly become intramolecular disulfide bonds, but improperly become a large number of intermolecular disulfide bonds. Compared with the molecular designs of 1409 and 1410, in 1409, a glycine (Ins-G172) is inserted after P171, and the insertion of G172 can increase a space for the generation of intramolecular disulfide bonds between C170 and C173. For example, the substitution of the inserted amino acid G172 with A172 (the FGF21 mutant 1412) or S172 (the FGF21 mutant 1413) can lead to a similar technical effect, but a small number of incorrect dimers can still be generated. The FGF21 mutant 1411 is obtained by shifting a mutation after P171 to position 173 (SEQ ID NO: 7), and only a trace amount of incorrect dimers are generated in this mutant.

### Example 3 In vitro FAP cleavage test

FAP is a protease present *in vivo.* FAP can specifically cleave an amino acid between 171 and 172 of FGF21, such that a C-terminus of FGF21 injected into a body or present in a body is incomplete, and thus cannot bind to a receptor β-klotho in the body, resulting in a loss of an activity and tissue specificity. In this example, an *in vitro* FAP (purchased from ACROBiosystems, Item No. FAP-H5244) cleavage test was conducted to screen out FGF21 mutants that were not cleaved. The test was conducted according to the literature (http://dx.doi.org/10.1016/j.molmet.2016.07.003). Test results are shown in Table 2. Under the same experimental conditions, when the FGF21 mutant 1414 as a negative control is completely cleaved by FAP, the FGF21 mutant P171G (1407) is cleaved by about a half, but the mutants 1408 to 1413 (1410 is not detected) designed in the present disclosure are cleaved merely at a trace amount or are not cleaved.

**Table 2 FAP cleavage test results of FGF21 and mutants thereof**

| **FGF21 and mutants thereof** | **Percentage of fragments produced due to cleavage** |
|---|---|
| 1407 | 46% |
| 1408 | < 2% |
| 1409 | 0 |
| 1410 | Undetected |
| 1411 | 0 |
| 1412 | 0 |
| 1413 | 0 |
| 1414 | 100% |

### Example 4 Test of binding of FGF21 mutants to a receptor β-Klotho

An extracellular domain of a human receptor β-Klotho (a Poly-His tag was fused at a C-terminus) was expressed and purified in the present disclosure, and a sequence of the protein was acquired from the Uniprot database. An affinity assay was conducted with Biacore-8K. Specifically, a human β-Klotho protein was immobilized on an NTA chip at a fixed amount of 1,000 RU with PBS (including 0.05% Tween-20) as a flow buffer. An FGF21 mutant was allowed to flow through the chip at different concentrations to determine the binding of the FGF21 mutant to β-Klotho. Final data were fitted using kinetics, and a dissociation constant KD was calculated. Specific affinity results are shown in Table 3. It can be seen that the mutants 1407 to 1413 (1410 is not tested) all exhibit a similar affinity for β-Klotho to the FGF21 control mutant 1414, indicating that an affinity of each mutant is not inhibited and the mutants have similar affinities.

**Table 3 Binding activities of FGF21 and mutants thereof to β-Klotho**

| **FGF21 and mutants thereof** | **Binding strength to β-Klotho (M)** |
|---|---|
| 1407 | 4.25E-08 |
| 1408 | 6.75E-08 |
| 1409 | 5.94E-08 |
| 1410 | Undetected |
| 1411 | 6.94E-08 |
| 1412 | 6.99E-08 |
| 1413 | 6.46E-08 |
| 1414 | 5.12E-08 |

### Example 5 Experiment of FGF21 and mutants thereof in β-Klotho-ERK cells

In order to further verify activities of the FGF21 and mutants thereof in the present disclosure, activation effects of the FGF21 and mutants thereof for an ERK signaling pathway were tested in HEK293 cells overexpressing β-Klotho (the cells were constructed by Pharmaron). Specific experimental steps were as follows: The HEK293 cells overexpressing β-Klotho were cultivated with a DMEM + 10% FBS + 1*PS medium until an exponential growth phase was reached, then 20 µL of a cell suspension was taken and added to a white 384-well plate, an appropriate amount of a medium was added to adjust to a cell density to 5,000/well, and the plate was incubated at 37°C and 5% CO₂. The FGF21 and mutants were subjected to 3-fold serial dilution in a medium starting from 3 µM/6 µM/15 µM, and a resulting dilution was added to the plate at 10 µL of a compound/well. After proteins to be tested were added, the cells were cultivated in a 37°C and 5% CO₂ incubator for 0.5 h. A resulting cell supernatant was removed, 16 µL of a cell lysis buffer was added, and the plate was shaken at room temperature for 30 min to 60 min. 4 µL of a premixed antibody solution prepared in a detection buffer was added, the plate was sealed with a sealing film and then incubated overnight at room temperature, and fluorescence emissions at 665 nm and 620 nm were read. An activity of a mutant was measured by comparing EC50 values of the mutant. Experimental results are shown in Table 4. The mutants 1407 to 1413 (1410 is not tested) have a very similar biological activity to the FGF21 control mutant 1414.

**Table 4 Activities of FGF21 and mutants thereof in cells**

| **FGF21 and mutants thereof** | **Activity** in β-Klotho-ERK cells (nM) |
|---|---|
| 1407 | 1.152 |
| 1408 | 1.484 |
| 1409 | 2.140 |
| 1410 | Undetected |
| 1411 | 0.999 |
| 1412 | 2.516 |
| 1413 | 2.093 |
| 1414 | 0.850 |

The preferred specific examples of the present disclosure are described in detail above.

## Claims

1. A fibroblast growth factor 21 (FGF21) mutant protein comprising an amino acid sequence obtained through the following: on an amino acid sequence of wild-type human FGF21,
two cysteine residues are introduced through substitution and/or addition at a position corresponding to one to three amino acids before and after the proline residue at position 171 and form a disulfide bond at the two cysteine residues, wherein the two cysteine residues to form the disulfide bond are separated by 2 or 3 amino acids.

2. The FGF21 mutant protein according to claim 1, wherein after being introduced through substitution or addition, the two cysteine residues are directly separated by 2 or 3 amino acids; or after being introduced through substitution or addition, the two cysteine residues are separated by 2 or 3 amino acids through addition of amino acid residues.

3. The FGF21 mutant protein according to claim 1, wherein
a. an amino acid residue at a position corresponding to one to three amino acids before position 171 is substituted with a cysteine residue;
b. an amino acid residue at a position corresponding to one to three amino acids after position 171 is substituted with a cysteine residue;
c. one or two amino acid residues is/are added between position 171 and the position before position 171 substituted with a cysteine residue, and/or one or two amino acid residues is/are added between the position 171 and the position after position 171 substituted with a cysteine residue.

4. The FGF21 mutant protein according to claim 3, wherein the one or two amino acid residues added is/are selected from glycine, alanine, or serine.

5. The FGF21 mutant protein according to claim 3, wherein
a. 170G is substituted with 170C;
b. 172S is substituted with 172C; and
c. one or two amino acid residues are inserted between 171P and 172C.

6. The FGF21 mutant protein according to claim 1, comprising the amino acid sequence selected from amino acid sequences shown in SEQ ID NOS: 5, 7, 8, 9, 11, 13, 14, 15, 17, 19, 20, 21, 23, 25, 26, and 27.

7. The FGF21 mutant protein according to claim 1, wherein 121N is substituted with 121Q and 168M is substituted with 168L; and/or an alanine, glycine, or serine residue is added before a histidine residue at position 1 of the amino acid sequence of the wild-type human FGF21.

8. The FGF21 mutant protein according to claim 1, wherein the FGF21 mutant protein has a chemical modification.

9. A nucleic acid encoding the FGF21 mutant protein according to any one of claims 1 to 7.

10. A vector carrying the nucleic acid according to claim 9.

11. A genetically-engineered cell carrying the vector according to claim 10.

12. A pharmaceutical composition comprising the FGF21 mutant protein according to any one of claims 1 to 8 and a pharmaceutically acceptable excipient.

13. The FGF21 mutant protein according to any one of claims 1 to 8 or the pharmaceutical composition according to claim 12 for use in a method of controlling blood glucose and blood lipid levels and reducing a body weight.

## Patentansprüche

1. Ein Fibroblasten-Wachstumsfaktor-21-(FGF21)-Mutantenprotein, umfassend eine Aminosäuresequenz, erhalten durch Folgendes: auf einer Aminosäuresequenz von Wildtyp-humanem FGF21,
werden zwei Cysteinreste durch Substitution und/oder Addition an einer Position eingeführt, die ein bis drei Aminosäuren vor oder nach dem Prolinrest an Position 171 entspricht, und bilden eine Disulfidbindung an den zwei Cysteinresten, wobei die zwei Cysteinreste zur Bildung der Disulfidbindung durch 2 oder 3 Aminosäuren voneinander getrennt sind.

2. FGF21-Mutantenprotein nach Anspruch 1, wobei nach der Einführung durch Substitution oder Addition die zwei Cysteinreste unmittelbar durch 2 oder 3 Aminosäuren voneinander getrennt sind; oder wobei nach der Einführung durch Substitution oder Addition die zwei Cysteinreste durch Addition von Aminosäureresten in einem Abstand von 2 oder 3 Aminosäuren voneinander angeordnet sind.

3. FGF21-Mutantenprotein nach Anspruch 1, wobei
a. ein Aminosäurerest an einer Position, die ein bis drei Aminosäuren vor Position 171 entspricht, durch einen Cysteinrest substituiert ist;
b. ein Aminosäurerest an einer Position, die ein bis drei Aminosäuren nach Position 171 entspricht, durch einen Cysteinrest substituiert ist;
c. ein oder zwei Aminosäurereste zwischen Position 171 und derjenigen Position, die ein bis drei Aminosäuren vor Position 171 liegt und durch einen Cysteinrest substituiert ist, und/oder ein oder zwei Aminosäurereste zwischen Position 171 und derjenigen Position, die ein bis drei Aminosäuren nach Position 171 liegt und durch einen Cysteinrest substituiert ist, hinzugefügt sind.

4. FGF21-Mutantenprotein nach Anspruch 3, wobei der/die hinzugefügte(n) ein oder zwei Aminosäurerest(e) ausgewählt ist/sind aus Glycin, Alanin oder Serin.

5. FGF21-Mutantenprotein nach Anspruch 3, wobei
a. 170G durch 170C substituiert ist;
b. 172S durch 172C substituiert ist; und
c. ein oder zwei Aminosäurereste zwischen 171P und 172C eingefügt sind.

6. FGF21-Mutantenprotein nach Anspruch 1, umfassend die Aminosäuresequenz ausgewählt aus Aminosäuresequenzen, gezeigt in SEQ ID Nrn.: 5, 7, 8, 9, 11, 13, 14, 15, 17, 19, 20, 21, 23, 25, 26 und 27.

7. FGF21-Mutantenprotein nach Anspruch 1, wobei 121N durch 121Q substituiert ist und 168M durch 168L substituiert ist; und/oder ein Alanin-, Glycin- oder Serinrest vor einem Histidinrest an Position 1 der Aminosäuresequenz des Wildtyp-humanen FGF21 hinzugefügt ist.

8. FGF21-Mutantenprotein nach Anspruch 1, wobei das FGF21-Mutantenprotein eine chemische Modifikation aufweist.

9. Nukleinsäure, die das FGF21-Mutantenprotein nach einem der Ansprüche 1 bis 7 kodiert.

10. Vektor, der die Nukleinsäure nach Anspruch 9 enthält.

11. Gentechnisch modifizierte Zelle, die den Vektor nach Anspruch 10 enthält.

12. Pharmazeutische Zusammensetzung, umfassend das FGF21-Mutantenprotein nach einem der Ansprüche 1 bis 8 und einen pharmazeutisch verträglichen Hilfsstoff.

13. FGF21-Mutantenprotein nach einem der Ansprüche 1 bis 8 oder pharmazeutische Zusammensetzung nach Anspruch 12 zur Verwendung in einem Verfahren zur Kontrolle von Blutzucker- und Blutfettwerten und zur Reduktion eines Körpergewichts.

## Revendications

1. Protéine mutante du facteur de croissance des fibroblastes 21 (FGF21) comprenant une séquence d'acides aminés obtenue comme suit : sur une séquence d'acides aminés du FGF21 humain de type sauvage,
deux résidus cystéine sont introduits par substitution et/ou addition en une position correspondant à un à trois acide(s) aminé(s) avant ou après le résidu proline en position 171 et forment une liaison disulfure au niveau des deux résidus cystéine, dans laquelle les deux résidus cystéine destinés à former la liaison disulfure sont séparés par 2 ou 3 acides aminés.

2. Protéine mutante FGF21 selon la revendication 1, dans laquelle, après avoir été introduits par substitution ou addition, les deux résidus cystéine sont directement séparés par 2 ou 3 acides aminés ; ou après avoir été introduits par substitution ou addition, les deux résidus cystéine sont séparés par 2 ou 3 acides aminés par addition de résidus d'acides aminés.

3. Protéine mutante FGF21 selon la revendication 1, dans laquelle
a. un résidu d'acide aminé en une position correspondant à un à trois acide(s) aminé(s) avant la position 171 est substitué par un résidu cystéine ;
b. un résidu d'acide aminé en une position correspondant à un à trois acide(s) aminé(s) après la position 171 est substitué par un résidu cystéine ;
c. un ou deux résidu(s) d'acides aminés est/sont ajouté(s) entre la position 171 et la position avant la position 171 substituée par un résidu cystéine, et/ou un ou deux résidu(s) d'acides aminés est/sont ajouté(s) entre la position 171 et la position après la position 171 substituée par un résidu cystéine.

4. Protéine mutante FGF21 selon la revendication 3, dans laquelle le ou les deux résidu(s) d'acides aminés ajouté(s) est/sont choisi(s) parmi la glycine, l'alanine ou la sérine.

5. Protéine mutante FGF21 selon la revendication 3, dans laquelle
a. 170G est substitué par 170C ;
b. 172S est substitué par 172C ; et
c. un ou deux résidu(s) d'acides aminés est/sont inséré(s) entre 171P et 172C.

6. Protéine mutante FGF21 selon la revendication 1, comprenant la séquence d'acides aminés choisie parmi les séquences d'acides aminés représentées dans les SEQ ID N° : 5, 7, 8, 9, 11, 13, 14, 15, 17, 19, 20, 21, 23, 25, 26 et 27.

7. Protéine mutante FGF21 selon la revendication 1, dans laquelle 121N est substitué par 121Q et 168M est substitué par 168L ; et/ou un résidu alanine, glycine ou sérine est ajouté avant un résidu histidine en position 1 de la séquence d'acides aminés du FGF21 humain de type sauvage.

8. Protéine mutante FGF21 selon la revendication 1, dans laquelle la protéine mutante FGF21 présente une modification chimique.

9. Acide nucléique codant pour la protéine mutante FGF21 selon l'une quelconque des revendications 1 à 7.

10. Vecteur portant l'acide nucléique selon la revendication 9.

11. Cellule génétiquement modifiée portant le vecteur selon la revendication 10.

12. Composition pharmaceutique comprenant la protéine mutante FGF21 selon l'une quelconque des revendications 1 à 8 et un excipient pharmaceutiquement acceptable.

13. Protéine mutante FGF21 selon l'une quelconque des revendications 1 à 8 ou la composition pharmaceutique selon la revendication 12 pour utilisation dans un procédé de régulation de la glycémie et des taux de lipides sanguins et de réduction d'un poids corporel.
